(19) Europäisches Patentamt
European Patent Office
Office européen des brevets

(11) **EP 4 434 346 A1**

(12) **EUROPEAN PATENT APPLICATION**
published in accordance with Art. 153(4) EPC

(43) Date of publication:
**25.09.2024 Bulletin 2024/39**

(21) Application number: **22895154.7**

(22) Date of filing: **12.07.2022**

(51) International Patent Classification (IPC):
**A01N 65/06** (2009.01)     **A01N 31/02** (2006.01)
**A01N 61/00** (2006.01)     **A01N 63/10** (2020.01)
**A01P 1/00** (2006.01)      **A61K 35/10** (2015.01)
**A61K 36/15** (2006.01)     **A61P 31/12** (2006.01)

(52) Cooperative Patent Classification (CPC):
**A01N 31/02; A01N 61/00; A01N 63/10;
A01N 65/06; A01P 1/00; A61K 35/10; A61K 36/15;
A61P 31/12**

(86) International application number:
**PCT/JP2022/027429**

(87) International publication number:
**WO 2023/089865 (25.05.2023 Gazette 2023/21)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **16.11.2021   JP 2021186350**

(71) Applicant: **Hanic White Labo Co., Ltd.
Tokyo 102-0082 (JP)**

(72) Inventors:
• **URAI, Kaoruko
  Tokyo 102-0082 (JP)**
• **WADA, Sachiko
  Chiba-shi, Chiba 260-0856 (JP)**
• **URAI, Yasutaka
  Tokyo 102-0082 (JP)**

(74) Representative: **Hasegawa, Kan
Patentanwaltskanzlei Hasegawa
Untere Hauptstraße 56
85354 Freising (DE)**

(54) **ANTIVIRAL AGENT**

(57)     Provided is a novel antiviral agent. An antiviral agent containing at least one of rosin, copal, and shellac.

EP 4 434 346 A1

**Description**

**TECHNICAL FIELD**

The present invention relates to an antiviral agent.

**BACKGROUND ART**

In recent years, new viral infections have become a threat. For example, SARS-CoV-2 (novel coronavirus) was discovered in 2019 and has spread worldwide. Infectious diseases caused by severe acute respiratory syndrome coronavirus (SARS-CoV-1) prevail in 2002, and infectious diseases caused by Middle East respiratory syndrome coronavirus (MERS-CoV) prevail in 2012. Other than, infectious diseases caused by noroviruses, influenza viruses, and the like are regularly prevalent.
As one of countermeasures against virus infection, it is possible to reduce the number of attached viruses. For example, countermeasures against viruses attached to hands, fingers, and the like include washing away and alcohol disinfection. Examples of countermeasures against viruses attached to articles include disinfection with hot water, alcohol, or the like.
A method of reducing the number of viruses attached to an article by imparting antiviral properties to the article is also used. For example, WO 2019/045110 A discloses an antiviral composition comprising a resin, monovalent copper compound particles coated with a dispersant, and a hydrophilic compound.

**SUMMARY OF INVENTION**

Technical Problem

An object of the present invention is to provide a novel antiviral agent.

Solution to Problem

The present inventors have conducted intensive studies to solve the above problems. As a result, the present inventors have found that the above problems can be solved by using at least one of rosin, copal, and shellac, thereby completing the present invention.
That is, according to an aspect of the present invention, there is provided an antiviral agent containing at least one of rosin, copal, and shellac.

Advantageous Effects of Invention

According to the present invention, it is possible to provide a novel antiviral agent.

**DESCRIPTION OF EMBODIMENTS**

Hereinafter, embodiments according to an aspect of the present invention will be described. The present invention is not limited only to the following embodiments.
In the present specification, the phrase "X to Y" indicating a range means "X or more and Y or less". Unless otherwise specified, operations and measurements of physical properties and the like are measured under the conditions of room temperature (20 to 25°C)/relative humidity of 40 to 50% RH.

<Antiviral agent>

An aspect of the present invention is an antiviral agent containing at least one of rosin, copal, and shellac.
As a result of studies by the present inventors, it has been found that antiviral properties can be exhibited even when rosin, copal, and shellac are used alone or in combination (see Examples and Test Examples described below). That is, an antiviral agent containing rosin, copal, and shellac alone or a combination thereof can be provided.
In the present specification, the term "antiviral agent" means an agent capable of reducing a viral infectivity titer (the number of viruses having cell infectivity) by coming into contact with a virus. Specifically, the viral infectivity titer is measured by a $TCID_{50}$ measurement method. The reduction in viral infectivity titer means that a reduction rate calculated by the method described in Examples is 50% or more, preferably 70% or more.
In the present specification, the term "antiviral properties" means to reduce a viral infectivity titer (the number of viruses having cell infectivity).

The virus to be a subject of the antiviral agent according to the present invention may be an enveloped virus or a non-enveloped virus. Examples of the enveloped virus include coronaviruses (including SARS-CoV-2), influenza viruses, herpesviruses, rubella viruses, and the like. Examples of the non-enveloped virus include noroviruses, rotaviruses, and the like. The virus to be a subject is preferably an enveloped virus, and more preferably a coronavirus.

Rosin, copal (copal resin, copaiba), and shellac used in the antiviral agent according to the present invention are natural resins. Rosin and copal are plant-derived resins, and shellac is an animal-derived resin. Rosin and shellac are listed in List of Existing Food Additives (The Japan Food Chemical Research Foundation, revised on February 26, 2020). Although copal is deleted from List of Existing Food Additives (2011, The Minister of Health, Labour and Welfare Notification No. 158), it has been reported that "No toxicity was considered to have an adverse effect on human health to the extent that it was used as an additive" (Research on the safety re-evaluation of existing additives of The Japan Food Chemical Research Foundation (research in 2009)). Copal (copaiba) is described in List of original source plants and animals of natural flavoring agents (The Japan Food Chemical Research Foundation).

The types of rosin, copal, and shellac are not particularly limited, but those used in use applications such as food, medical, and cosmetics are preferable.

In a preferred embodiment, the antiviral agent according to the present invention contains rosin and shellac. By combining rosin and shellac, as compared with the case of using rosin or shellac alone, the drying time for forming a membrane can be shortened. The duration of the membrane can be further lengthened.

When the antiviral agent according to the present invention contains rosin and shellac, the content of shellac is, for example, 0.02 to 2.0 parts by mass, preferably 0.05 to 1.8 parts by mass, and more preferably 0.07 to 1.5 parts by mass, with respect to 1.0 part by mass of rosin.

In a preferred embodiment, the antiviral agent according to the present invention contains rosin, copal, and shellac. By combining rosin, copal, and shellac, as compared with the case of using rosin, copal, or shellac alone, the drying time for forming a membrane can be further shortened (for example, about 10 to 15 seconds), and a membrane to be formed can be made transparent. A membrane to be formed is glossy and can maintain its glossiness for a long time.

When the antiviral agent according to the present invention contains rosin, copal, and shellac, the content of copal is, for example, 0.02 to 2.0 parts by mass, preferably 0.1 to 1.5 parts by mass, and more preferably 0.5 to 1.2 parts by mass, with respect to 1.0 part by mass of rosin. The content of shellac is, for example, 0.02 to 2.0 parts by mass, preferably 0.05 to 1.8 parts by mass, and more preferably 0.07 to 1.5 parts by mass, with respect to 1.0 part by mass of rosin.

As the rosin, the copal, and the shellac according to the present invention, commercially available products can be used.

<Antiviral composition>

An aspect of the present invention is an antiviral composition containing the above-described antiviral agent and a solvent. The antiviral composition according to the present invention can further contain a vegetable oil, other components, and the like as necessary.

The solvent is not particularly limited as long as it can dissolve rosin, copal, and/or shellac. Examples of the solvent include alcohols such as methanol, ethanol, 2-propanol (isopropyl alcohol), and n-butanol; and esters such as ethyl acetate and butyl acetate. The solvent is preferably an alcohol, and more preferably ethanol (particularly, absolute ethanol).

In the antiviral composition, the content of the antiviral agent can be appropriately adjusted depending on the use application. The content of the antiviral agent is, for example, 1 to 50 mass% with respect to the total mass of the antiviral composition. When the antiviral composition is used for forming an antiviral membrane, the content of the antiviral agent is preferably 3 to 30 mass% and more preferably 5 to 15 mass%, with respect to the total mass of the antiviral composition.

In the antiviral composition, the content of the solvent is not particularly limited, and is, for example, 50 to 95 mass% and preferably 70 to 90 mass% with respect to the total mass of the antiviral composition.

In one embodiment, the antiviral composition according to the present invention further contains a vegetable oil. Examples of the vegetable oil include olive oil, camellia oil, sunflower oil, safflower oil, macadamia nut oil, rapeseed oil, almond oil, castor oil, jojoba oil, rose oil, and the like.

In the antiviral composition, the content of the vegetable oil is, for example, 1 to 5 mass% with respect to the total mass of the antiviral composition.

In the antiviral composition according to the present invention, examples of the other components include flavoring agents (excluding vegetable oils), polymer compounds (excluding rosin, copal, and shellac), and the like.

Examples of the flavoring agent include synthetic flavoring agents of terpenes such as ketone, aldehyde, limonene, linalool, citronellol, menthol, and geraniol, natural flavoring agents, flavoring agents used as food additives, and the like.

Examples of the polymer compound include polyvinyl alcohol, polyvinyl pyrrolidone, hydroxypropyl cellulose, a carboxyvinyl polymer, and an acrylic resin (a resin containing a constituent unit derived from a (meth)acrylate compound).

In the antiviral composition, the content of the other components is, for example, 5 mass% or less and more preferably 3 mass% or less (lower limit value: 0 mass%), with respect to the total mass of the antiviral composition.

When the antiviral composition contains a vegetable oil and a flavoring agent, the total content of the vegetable oil and the flavoring agent is, for example, 5 mass% or less and preferably 3 to 5 mass% with respect to the total mass of the antiviral composition. When the total content of the vegetable oil and the flavoring agent is in such a range, quick-drying property when a membrane is formed can be maintained.

A method for producing the antiviral composition according to the present invention is not particularly limited, and for example, the antiviral composition can be obtained by mixing at least one of rosin, copal, and shellac contained in the antiviral agent, a solvent, and, as necessary, a vegetable oil, other components, and the like.

<Use application>

The antiviral agent and the antiviral composition according to the present invention can be used in use applications for imparting antiviral properties to a subject. That is, one embodiment of the present invention relates to a method for imparting antiviral properties to a subject, the method including: allowing the above-described antiviral agent or antiviral composition to be contained in a subject or to be attached to a surface of the subject.

The subject to which antiviral properties are imparted will be described in an antiviral membrane described below.

Since rosin, copal, and shellac have the property of forming a membrane, these components are suitable for membrane formation. That is, one embodiment of the present invention relates to an antiviral agent or antiviral composition used for membrane formation.

<Antiviral membrane>

One embodiment of the present invention relates to an antiviral membrane formed by using the above-described antiviral composition. The antiviral membrane can be formed, for example, by applying the antiviral composition to a subject and curing the antiviral composition.

Examples of the subject to which the antiviral composition is applied include metals (such as aluminum, iron, and gold), ceramics, glass, fibers, nonwoven fabrics, films, plastic, rubber, paper, wood, stones; skin, oral mucosa, and teeth of animals (such as humans); food (solid), and the like.

A method of applying the antiviral composition is not particularly limited, and examples thereof include brush coating, rod coating, finger coating, a spin coating method, a dip coating method, a spray coating method, and the like.

The curing conditions after application are not particularly limited, and may be time and temperature at which a membrane is formed. The curing time is, for example, about 15 to 30 seconds. The curing temperature may be room temperature (15 to 30°C), and heating may be performed to evaporate the solvent as necessary.

<Antiviral article>

One embodiment of the present invention relates to an antiviral article having the antiviral membrane at least a part of a surface (for example, a part to be touched by a human).

Examples of the article include articles formed from metals (such as aluminum, iron, and gold), ceramics, glass, fibers, nonwoven fabrics, films, plastic, rubber, paper, wood, stones, and the like. Examples of the article include doorknobs, straps, toys, containers, furniture, clothing, tableware, and the like.

A method for producing the antiviral article is not particularly limited, and the antiviral article can be formed by applying the antiviral composition to an article and curing the antiviral composition. The coating method and the curing conditions are the same as those of the antiviral membrane, and thus the description thereof will be omitted.

<Embodiments>

Hereinafter, embodiments according to the present invention will be exemplified.

[1] An antiviral agent containing at least one of rosin, copal, and shellac.
[2] The antiviral agent according to [1], containing the rosin and the shellac.
[3] The antiviral agent according to [2], containing 0.02 to 2.0 parts by mass of the shellac with respect to 1.0 part by mass of the rosin.

[4] The antiviral agent according to any one of [1] to [3], containing the rosin, the copal, and the shellac.

[5] The antiviral agent according to [4], containing 0.02 to 2.0 parts by mass of the copal and 0.02 to 2.0 parts by mass of the shellac with respect to 1.0 part by mass of the rosin.

[6] An antiviral composition containing the antiviral agent according to any one of [1] to [5] and a solvent.

[7] The antiviral composition according to [6], further containing a vegetable oil.

[8] The antiviral composition according to [6] or [7], which is used for membrane formation.

[9] An antiviral membrane formed by using the antiviral composition according to [8].

[10] An antiviral article containing the antiviral membrane according to [9] on at least a part of a surface.

[11] An antiviral agent consisting of at least one of copal and shellac.

[12] An antiviral agent consisting of at least one of copal and shellac, and rosin.

[13] The antiviral agent according to [12], consisting of the rosin and the shellac.

[14] The antiviral agent according to [13], containing 0.02 to 2.0 parts by mass of the shellac with respect to 1.0 part by mass of the rosin.

[15] The antiviral agent according to any one of [12] to [14], consisting of the rosin, the copal, and the shellac.

[16] The antiviral agent according to [15], containing 0.02 to 2.0 parts by mass of the copal and 0.02 to 2.0 parts by mass of the shellac with respect to 1.0 part by mass of the rosin.

[17] An antiviral composition containing the antiviral agent according to any one of [11] to [16] and a solvent, the antiviral composition being used for imparting antiviral properties to a subject.

[18] The antiviral composition according to [17], further containing a vegetable oil.

[19] The antiviral composition according to [17] or [18], which is used for membrane formation.

[20] An antiviral membrane formed by using the antiviral composition according to [19].

[21] An antiviral article comprising the antiviral membrane according to [20] on at least a part of a surface.

[22] A method for imparting antiviral properties to a subject, the method including: allowing the antiviral agent according to any one of [1] to [5] and [11] to [16] or the antiviral composition according to any one of [6], [7], [17], and [18] to be contained in a subject or to be attached to at least a part of the subject.

[23] Use of at least one resin selected from the group consisting of rosin, copal, and shellac as an antiviral agent.

[24] Use of the antiviral agent according to any one of [1] to [5] and [11] to [16] or the antiviral composition according to any one of [6], [7], [17], and [18] for reducing a viral infectivity titer.

Examples

The effects of the present invention will be described with the following Examples and Comparative Examples. However, the technical scope of the present invention is not limited only to the following Examples.

[Preparation example of antiviral composition]

Compositions of Examples were prepared by the following method. The blending amount of each raw material in each Example is shown in Table 1 below. The blending amount of each component in each Example is shown in Table 2 below.

(Example 1)

A composition of a uniform solution was obtained by thoroughly mixing and dissolving 13.0 mass% of gum rosin (manufactured by LAWTER ARGENTINA S.A.), 43.3 mass% of a 30 mass% copal ethanol solution, 2.0 mass% of LACCOAT 50EDS (50 mass% shellac ethanol solution) (manufactured by THE JAPAN SHELLAC INDUSTRIES, LTD.), and 41.7 mass% of specific alcohol 99 degrees, first grade, fermented, unmodified (absolute ethanol) (manufactured by AMAKSU CHEMICAL INDUSTRIES) by a stirrer.

The 30 mass% copal ethanol solution was obtained by the following method.

An unpurified copal resin (solid) was dissolved in specific alcohol 99 degrees, first grade, fermented, unmodified (absolute ethanol) (manufactured by AMAKSU CHEMICAL INDUSTRIES) and then filtered with a 200 mesh sheet, and the obtained filtrate was tested according to Testing methods for paint components (JIS K 5601-1-2:2008) to calculate a copal concentration. Specific alcohol 99 degrees, first grade, fermented, unmodified (absolute ethanol) (manufactured by AMAKSU CHEMICAL INDUSTRIES) was added so that the filtrate was a 30 mass% copal solution, the concentration was adjusted, and then the solution was sufficiently stirred to obtain a uniform solution. The obtained solution was stored in a sealed container and allowed to stand still, and the obtained supernatant was used as a 30 mass% copal ethanol solution.

(Example 2)

A composition of a uniform solution was obtained by thoroughly mixing and dissolving 10.0 mass% of gum rosin (manufactured by LAWTER ARGENTINA S.A.), 20.0 mass% of LACCOAT 50EDS (50 mass% shellac ethanol solution) (manufactured by THE JAPAN SHELLAC INDUSTRIES, LTD.), and 70.0 mass% of specific alcohol 99 degrees, first grade, fermented, unmodified (absolute ethanol) (manufactured by AMAKSU CHEMICAL INDUSTRIES) by a stirrer.

(Example 3)

A composition of a uniform solution was obtained by thoroughly mixing and dissolving 13.0 mass% of gum rosin (manufactured by LAWTER ARGENTINA S.A.), 43.3 mass% of a 30 mass% copal ethanol solution, 2.0 mass% of LACCOAT 50EDS (50 mass% shellac ethanol solution) (manufactured by THE JAPAN SHELLAC INDUSTRIES, LTD.), 2.0 mass% of olive oil refinement (manufactured by DSP Gokyo Food & Chemical Co., Ltd.), and 39.7 mass% of specific alcohol 99 degrees, first grade, fermented, unmodified (absolute ethanol) (manufactured by AMAKSU CHEMICAL INDUSTRIES) by a stirrer.

(Example 4)

A composition of a uniform solution was obtained by thoroughly mixing and dissolving 13.0 mass% of gum rosin (manufactured by LAWTER ARGENTINA S.A.) and 87.0 mass% of specific alcohol 99 degrees, first grade, fermented, unmodified (absolute ethanol) (manufactured by AMAKSU CHEMICAL INDUSTRIES) by a stirrer.

(Example 5)

A composition of a uniform solution was obtained by thoroughly mixing and dissolving 43.3 mass% of a 30 mass% copal ethanol solution and 56.7 mass% of specific alcohol 99 degrees, first grade, fermented, unmodified (absolute ethanol) (manufactured by AMAKSU CHEMICAL INDUSTRIES) by a stirrer.

(Example 6)

A composition of a uniform solution was obtained by thoroughly mixing and dissolving 20.0 mass% of LACCOAT 50EDS (50% shellac ethanol solution) (manufactured by THE JAPAN SHELLAC INDUSTRIES, LTD.), and 80.0 mass% of specific alcohol 99 degrees, first grade, fermented, unmodified (absolute ethanol) (manufactured by AMAKSU CHEMICAL INDUSTRIES) by a stirrer.

[Table 1]

| (Table 1) Raw material blending table | | | | | | |
|---|---|---|---|---|---|---|
| Sample No. | Blending ratio (mass%) of raw material | | | | | |
| | Gum rosin | Copal solution | Laccoat | Vegetable oil | Ethanol | Total |
| Example 1 | 13.0 | 43.3 | 2.0 | 0.0 | 41.7 | 100 |
| Example 2 | 10.0 | 0.0 | 20.0 | 0.0 | 70.0 | 100 |
| Example 3 | 13.0 | 43.3 | 2.0 | 2.0 | 39.7 | 100 |
| Example 4 | 13.0 | 0.0 | 0.0 | 0.0 | 87.0 | 100 |
| Example 5 | 0.0 | 43.3 | 0.0 | 0.0 | 56.7 | 100 |
| Example 6 | 0.0 | 0.0 | 20.0 | 0.0 | 80.0 | 100 |
| Laccoat: LACCOAT 50EDS<br>Vegetable oil: olive oil refinement<br>Ethanol: specific alcohol 99 degrees, first grade, fermented, unmodified (absolute ethanol) | | | | | | |

[Table 2]

| (Table 2) Component blending table | | | | | | |
|---|---|---|---|---|---|---|
| Sample No. | Blanding ratio (mass%) of component | | | | | |
| | Rosin | Copal | Shellac | Vegetable oil | Ethanol | Total |
| Example 1 | 13 | 13 | 1 | 0 | 73 | 100 |
| Example 2 | 10 | 0 | 10 | 0 | 80 | 100 |
| Example 3 | 13 | 13 | 1 | 2 | 71 | 100 |
| Example 4 | 13 | 0 | 0 | 0 | 87 | 100 |
| Example 5 | 0 | 13 | 0 | 0 | 87 | 100 |
| Example 6 | 0 | 0 | 10 | 0 | 90 | 100 |

[Evaluation of antiviral properties]

In the following test, as absolute ethanol, specific alcohol 99 degrees, first grade, fermented, unmodified (absolute ethanol) (manufactured by AMAKSU CHEMICAL INDUSTRIES) was used. As cell maintenance cells, code M4655 Minimum Essential Medium Eagle (added with 0.2% gentamicin) manufactured by Sigma-Aldrich was used.

(Test Example 1)

- Test material

    1: Composition of Example 1
    2: Composition of Example 2

Each material was dissolved in an equal amount of absolute ethanol, and 1 mL (0.5 mL as each material) of the obtained solution was added dropwise to a plastic (polystyrene) piece cut into a size of 5 cm on each side, and then applied with a bacteria spreader so as to spread uniformly. Thereafter, drying was performed for about 3 hours under the open condition and the ethanol component was evaporated to obtain a test piece.

A coating film formed by using the composition of Example 1 had high transparency. A coating film formed by using the composition of Example 2 was transparent.

As a control group (Comparative Example 1), a test piece obtained by applying only absolute ethanol to a plastic (polystyrene) piece in the same manner and drying the resultant was used.

- Group setting

[Table 3]

| | | | Repeat number |
|---|---|---|---|
| Group | Analyte | Reaction time (hr) | Virus |
| Control | - | 0, 6 | 1 |
| Test 1 | Example 1 | 6 | 1 |
| Test 2 | Example 2 | 6 | 1 |

(Table 3)

- Preparation of test virus solution

Test virus: Porcine epidemic diarrhea (PED) virus strain P-5V
Host cell: vero cell (established cell line derived from kidney epithelium of African green monkey)

(1) The test virus was inoculated into vero cell sheets;
(2) After adsorption at 37°C for 1 hour, the inoculated virus solution was removed and washing was performed twice with sterile PBS;
(3) A cell maintenance medium was added, and the cells were cultured at 37°C in 5% $CO_2$;
(4) The culture supernatant was recovered when about 70 to 80% of cell degeneration was observed; and
(5) The recovered culture supernatant was centrifuged at 3000 rpm for 30 minutes, and the centrifuged supernatant was dispensed and stored at -70°C or lower to obtain a test virus solution.

- Inoculation of test virus solution and measurement of virus titer (viral infectivity titer)

Before performing the test, the test material-coated test piece subjected to the same treatment as the test method described below was washed out with 10 mL of the cell maintenance medium, and then further subjected to 10-fold serial dilution, and each diluted solution was inoculated into the cultured cells and cultured at 37°C in 5% $CO_2$ for 5 days. When the cultured cells did not show a normal shape, it was determined that there was cytotoxicity due to the material, and in this test, dilution ratios at which cytotoxicity was confirmed were excluded from the test.

As a result, since cytotoxicity was not confirmed in the washing solution stock solution, the detection limit in this test was set to $10^{0.5}$ $TCID_{50}$/mL as the concentration in the washing solution.

(1) 0.2 mL of a cell maintenance medium was added dropwise to the test pieces of the control group and the test group 18 hours before the start of the test, and the test pieces were stored in a state of being covered with a sterilized film (polyethylene film);
(2) The coating film of the test piece after the lapse of the storage was peeled off, 0.2 mL of the test virus solution was added, the coating film was covered again, the test piece was placed in a sealed container so as not to be dried, and the reaction was started;
(3) The test piece was allowed to stand still for 6 hours at room temperature according to the test settings to obtain a reaction time;
(4) After the lapse of the sensitization time, the whole coating film was transferred to a sterilized bag, 10 mL of a cell maintenance medium was added, and the resultant was thoroughly mixed to wash out the virus;
(5) The washing solution was further subjected to 10-fold serial dilution with a cell maintenance medium, and the diluted solution was inoculated into cultured cells of a 96-well microplate and cultured at 37°C in the presence of 5% $CO_2$ gas for 5 days; and
(6) From the presence or absence of CPE (cytopathic effect) after culture or the presence or absence of 1% chicken hemagglutination reaction of the culture supernatant, the virus titer (viral infectivity titer) ($TCID_{50}$) was measured.

- Evaluation

In the test results, the reduction rate (%) of the test group with respect to the control group was calculated for each test time point, and the effect was confirmed.

In this test, the reduction rate was calculated by the following equation. The results are shown in Table 4 below.

[Mathematical Formula 1]

$$\text{Reduction rate (\%)} = \frac{\text{Control group} - \text{Test group}}{\text{Control group}} \times 100$$

[Table 4]

| (Table 4)PED virus test results | Viral infectivity titer (TCID$_{50}$/test piece) | | Reduction rate (%) |
|---|---|---|---|
| | At start of test | After 6 hours | |
| Test group 1 (Example 1) | | $10^{3.9}$ (7900) | 75.3 |
| Test group 2 (Example 2) | $10^{5.5}$ | $10^{3.1}$ (1300) | 95.9 |
| Control group (Comparative Example 1) | | $10^{4.5}$ (32000) | |

As shown in Table 4, the viral infectivity titer was $10^{5.5}$ (TCID$_{50}$/test piece) at the start of the test.

After 6 hours, the natural attenuation was observed in the control group to have a viral infectivity titer of $10^{4.5}$ (TCID$_{50}$/test piece), the viral infectivity titer was $10^{3.1}$ (TCID$_{50}$/test piece), which was decreased by 75.3%, in Test Group 1, and the viral infectivity titer was $10^{3.1}$ (TCID$_{50}$/test piece), which was decreased by 95.9%, in Test Group 2.

From the above, it can be seen that in the test piece coated using the composition of each of Examples 1 and 2, a high virus-reducing effect on the PED virus can be obtained by contacting for 6 hours.

(Test Example 2)

- Test material

1: Composition of Example 2
2: Composition of Example 3

Each material was dissolved in an equal amount of absolute ethanol, and 1 mL (0.5 mL as each material) of the obtained solution was added dropwise to the bottom surface of a 25 cm$^2$ flat-bottom flask (made of polystyrene), and then applied so as to spread uniformly. Thereafter, drying was performed for about 12 hours without closing the cap and the ethanol component was evaporated to obtain a test piece. In Test Example 2, the drying time was set longer than that in Test Example 1 in order to remove the influence of ethanol.

In Test Example 2, and Test Examples 3 and 4 described below, a sealable flat-bottom flask was used in order to avoid infection hazard of the test virus (SARS-CoV-2). Since the test piece using the plastic (polystyrene) piece used in Test Example 1 and the test piece (bottom surface of the flat-bottom flask) using the flat flask used in Test Examples 2 to 4 can be regarded as the same polystyrene surface with a size of 25 cm$^2$, the experimental conditions of Test Examples 1 to 4 are consistent.

A coating film formed by using the composition of Example 2 was transparent. A coating film formed by using the composition of Example 3 had high transparency.

As a control group (Comparative Example 2), a test piece obtained by applying only absolute ethanol to the bottom surface of a 25 cm$^2$ flat-bottom flask in the same manner and drying the resultant was used.

- Group setting

[Table 5]

| (Table 5) | | | Repeat number | |
|---|---|---|---|---|
| Group | Analyte | Reaction time (hr) | | Virus |
| Control | - | 0, 6 | | 1 |
| Test 1 | Example 1 | 6 | | 1 |
| Test 2 | Example 2 | 6 | | 1 |

- Preparation of test virus solution

Test virus: Severe acute respiratory syndrome coronavirus 2 (SARS-CoV-2)
Host cell: vero cell (established cell line derived from kidney epithelium of African green monkey)

The test virus was a human-derived isolate. A virus strain was used which was separated and cultured from human saliva using vero cells and then subjected to confirmation of amplification of SARS-CoV-2 genes (officially announced method of The Minister of Health, Labour and Welfare) using real-time PCR.
(1) The test virus was inoculated into vero cell sheets;
(2) After adsorption at 37°C for 1 hour, the inoculated virus solution was removed and washing was performed twice with sterile PBS;
(3) A cell maintenance medium was added, and the cells were cultured at 37°C in 5% $CO_2$;
(4) The culture supernatant was recovered when about 70 to 80% of cell degeneration was observed; and
(5) The recovered culture supernatant was centrifuged at 3000 rpm for 30 minutes, and the centrifuged supernatant was dispensed and stored at -70°C or lower to obtain a test virus solution.

- Inoculation of test virus solution and measurement of virus titer (viral infectivity titer)

Before performing the test, the test material-coated test piece subjected to the same treatment as the test method described below was washed out with 10 mL of the cell maintenance medium, and then further subjected to 10-fold serial dilution, and each diluted solution was inoculated into the cultured cells and cultured at 37°C in 5% $CO_2$ for 5 days. When the cultured cells did not show a normal shape, it was determined that there was cytotoxicity due to the material, and in this test, dilution ratios at which cytotoxicity was confirmed were excluded from the test.
As a result, since cytotoxicity was not confirmed in the washing solution stock solution, the detection limit in this test was set to $10^{0.5}$ $TCID_{50}$/mL as the concentration in the washing solution.
(1) 0.2 mL of a cell maintenance medium was added dropwise to the test pieces of the control group and the test group 18 hours before the start of the test, and the test pieces were stored in a state of being covered with a sterilized film (polyethylene film).
(2) The coating film of the test piece after the lapse of the storage was peeled off, 0.2 mL of the test virus solution was added, the film was covered again, the cap was closed such that the test piece was not dried, and the reaction was started.
(3) The test piece was allowed to stand still for 6 hours at room temperature according to the test settings to obtain a reaction time.
(4) After the lapse of the sensitization time, 10 mL of a cell maintenance medium was added into the flask, and the resultant was thoroughly mixed while the film was peeled off to wash out the virus.
(5) The washing solution was further subjected to 10-fold serial dilution with a cell maintenance medium, and each diluted solution was inoculated into cultured cells of a 96-well microplate and cultured at 37°C in the presence of 5% $CO_2$ gas for 5 days.
(6) From the presence or absence of CPE (cytopathic effect) after culture or the presence or absence of 1% chicken hemagglutination reaction of the culture supernatant, the virus titer (viral infectivity titer) ($TCID_{50}$) was measured.

- Evaluation

In the test results, the antiviral activity value and the reduction rate of the test group with respect to the control group were calculated for each test time point, and the effect was confirmed.
In this test, the antiviral activity value and the reduction rate (%) were calculated by the following equations. The results are shown in Table 6.

Antiviral activity value = Common logarith of viral infectivity titer in control group = Common logarithm of viral infectivity in test group

$$\text{Reduction rate (\%)} = \frac{\text{Control group} - \text{Test group}}{\text{Control group}} \times 100$$

[Table 6]

| (Table 6) SARS-CoV-2 test results | | | |
|---|---|---|---|
| Group | Viral infectivity titer (TCID$_{50}$/test piece) | | Reduction rate (%) |
| | At start of test | After 6 hours | |
| Test group 1 (Example 2) | $10^{5.7}$ | $10^{3.3}$ | 98.4 |
| Test group 2 (Example 3) | | $10^{3.5}$ | 97.5 |
| Control group (Comparative Example 2) | | $10^{5.1}$ | - |

As shown in Table 6, the viral infectivity titer was $10^{5.7}$ (TCID$_{50}$/test piece) at the start of the test.

After 6 hours, the natural attenuation was observed in the control group to have a viral infectivity titer of $10^{5.1}$ (TCID$_{50}$/test piece), the antiviral activity value was 1.8 and the reduction rate was 98.4% in Test Group 1 at $10^{3.3}$ (TCID$_{50}$/test piece), and the antiviral activity value was 1.6 and the reduction rate was 97.5% in Test Group 2 at $10^{3.5}$ (TCID$_{50}$/test piece) .

From the above, it can be seen that in the test piece coated using the composition of each of Examples 2 and 3, a high virus-reducing effect on SARS-CoV-2 can be obtained by contacting for 6 hours.

(Test Example 3)

- Test material

1: The composition material of Example 3 was dissolved in an equal amount of absolute ethanol, and 1 mL (0.5 mL as the material) of the obtained solution was added dropwise to the bottom surface of a 25 cm$^2$ flat-bottom flask (made of polystyrene), and then applied so as to spread uniformly. Thereafter, drying was performed for about 12 hours without closing the cap and the ethanol component was evaporated to obtain a test piece. In Test Example 3, the drying time was set longer in order to remove the influence of ethanol.

A coating film formed by using the composition of Example 3 had high transparency.

As a control group (Comparative Example 3), a test piece obtained by applying only absolute ethanol to the bottom surface of a 25 cm$^2$ flat-bottom flask in the same manner and drying the resultant was used.

- Group setting

[Table 7]

| (Table 7) | | | |
|---|---|---|---|
| Group | Analyte | Reaction time (hr) | Repeat number |
| | | | Virus |
| Control | - | 0,2 | 1 |
| Test | Example 3 | 2 | 1 |

- Preparation of test virus solution

A test virus solution was prepared in the same manner as in Test Example 2.

- Inoculation of test virus solution and measurement of virus titer (viral infectivity titer)

Before performing the test, the test material-coated test piece subjected to the same treatment as the test method described below was washed out with 10 mL of the cell maintenance medium, and then further subjected to 10-fold serial dilution, and each diluted solution was inoculated into the cultured cells and cultured at 37°C in 5% $CO_2$ for 5 days. When the cultured cells did not show a normal shape, it was determined that there was cytotoxicity due to the material, and in this test, dilution ratios at which cytotoxicity was confirmed were excluded from the test.
As a result, since cytotoxicity was not confirmed in the washing solution stock solution, the detection limit in this test was set to $10^{0.5}$ $TCID_{50}$/mL as the concentration in the washing solution.
(1) 0.2 mL of a cell maintenance medium was added dropwise to the test pieces of the control group and the test group 18 hours before the start of the test, and the test pieces were stored in a state of being covered with a sterilized film (polyethylene film).
(2) The coating film of the test piece after the lapse of the storage was peeled off, 0.2 mL of the test virus solution was added, the film was covered again, the cap was closed such that the test piece was not dried, and the reaction was started.
(3) The test piece was allowed to stand still for 2 hours at room temperature according to the test settings to obtain a reaction time.
(4) After the lapse of the sensitization time, 10 mL of a cell maintenance medium was added into the flask, and the resultant was thoroughly mixed while the film was peeled off to wash out the virus.
(5) The washing solution was further subjected to 10-fold serial dilution with a cell maintenance medium, and each diluted solution was inoculated into cultured cells of a 96-well microplate and cultured at 37°C in the presence of 5% $CO_2$ gas for 5 days.
(6) From the presence or absence of CPE after culture, the virus titer (viral infectivity titer) ($TCID_{50}$) was measured.

- Evaluation

The antiviral activity value and the reduction rate (%) were calculated in the same manner as in Test Example 2.
The results are shown in Table 8.

[Table 8]

| (Table 8) SARS-CoV-2 test results | | | |
|---|---|---|---|
| Group | Viral infectivity titer ($TCID_{50}$/test piece) | | Reduction rate (%) |
| | At start of test | After 6 hours | |
| Test group (Example 3) | $10^{5.7}$ | $10^{3.7}$ | 98.4 |
| Control group (Comparative Example 3) | | $10^{5.5}$ | - |

As shown in Table 8, the viral infectivity titer was $10^{5.7}$ ($TCID_{50}$/test piece) at the start of the test.
After 2 hours, the slight natural attenuation was observed in the control group to have a viral infectivity titer of $10^{5.5}$ ($TCID_{50}$/test piece), and the antiviral activity value was 1.8 and the reduction rate was 98.4% in the test group at $10^{3.7}$ ($TCID_{50}$/test piece).
From the above, it can be seen that in the test piece coated using the composition of Example 3, a high virus-reducing effect on SARS-CoV-2 can be obtained by contacting for 2 hours.

(Test Example 4)

- Test material

1: Composition of Example 4
2: Composition of Example 5
3: Composition of Example 6

Each material was dissolved in an equal amount of absolute ethanol, and 1 mL (0.5 mL as each material) of the obtained solution was added dropwise to the bottom surface of a 25 cm$^2$ flat-bottom flask (made of polystyrene), and then applied so as to spread uniformly. Thereafter, drying was performed for about 12 hours without closing the cap and the ethanol component was evaporated to obtain a test piece. In Test Example 4, the drying time was set longer in order to remove the influence of ethanol.

A coating film formed by using the composition of each of Examples 4 to 6 was transparent.

As a control group (Comparative Example 4), a test piece obtained by applying only absolute ethanol to the bottom surface of a 25 cm$^2$ flat-bottom flask in the same manner and drying the resultant was used.

- Group setting

[Table 9]

(Table 9)

| Group | Analyte | Reaction time (hr) | Repeat number Virus |
|---|---|---|---|
| Control group | - | 0,2 | 1 |
| Test group 1 | Example 4 | 2 | 1 |
| Test group 2 | Example 5 | 2 | 1 |
| Test group 3 | Example 6 | 2 | 1 |

- Preparation of test virus solution

A test virus solution was prepared in the same manner as in Test Example 2.

- Inoculation of test virus solution and measurement of virus titer (viral infectivity titer)

The inoculation of the test virus solution and the measurement of the viral infectivity titer were performed by the same methods as in Test Example 3.

- Evaluation

The antiviral activity value and the reduction rate (%) were calculated in the same manner as in Test Example 2. The results are shown in Table 10.

[Table 10]

| (Table 10) SARS-CoV-2 test results | | | |
|---|---|---|---|
| Group | Viral infectivity titer (TCID$_{50}$/test piece) | | Reduction rate (%) |
| | At start of test | After 6 hours | |
| Test group 1 (Example 4) | 10$^{5.7}$ | 10$^{4.5}$ | 90.0 |
| Test group 2 (Example 5) | | 10$^{4.1}$ | 95.9 |
| Test group 3 (Example 6) | | 10$^{3.7}$ | 98.4 |
| Control group (Comparative Example 4) | | 10$^{5.5}$ | - |

As shown in Table 10, in the control group, the natural attenuation of the viral load was observed from the start of the test to 2 hours after the start of the test ($10^{5.7} \rightarrow 10^{5.5}$ TCID$_{50}$/test piece) .

The antiviral activity value was 1.0 and the reduction rate was 90.0% in Test Group 1 at $10^{4.5}$ (TCID$_{50}$/test piece) in 2 hours after the start, and the antiviral activity value was 1.4 and the reduction rate was 95.9% in Test Group 2 at $10^{4.1}$ (TCID$_{50}$/test piece). In Test Group 3, the antiviral activity value was 1.8 and the reduction rate was 98.4% at $10^{3.7}$ (TCID$_{50}$/test piece).

From the above, it can be seen that in the test piece coated using the composition of each of Examples 4 to 6, a high virus-reducing effect on SARS-CoV-2 can be obtained by contacting for 2 hours.

The present application is based on Japanese Patent Application No. 2021-186350 filed on November 16, 2021, the disclosure content of which is incorporated herein by reference in its entirety.

**Claims**

1. An antiviral agent consisting of at least one of copal and shellac.

2. An antiviral agent consisting of at least one of copal and shellac, and rosin.

3. The antiviral agent according to claim 2, consisting of the rosin and the shellac.

4. The antiviral agent according to claim 3, comprising 0.02 to 2.0 parts by mass of the shellac with respect to 1.0 part by mass of the rosin.

5. The antiviral agent according to any one of claims 2 to 4, consisting of the rosin, the copal, and the shellac.

6. The antiviral agent according to claim 5, comprising 0.02 to 2.0 parts by mass of the copal and 0.02 to 2.0 parts by mass of the shellac with respect to 1.0 part by mass of the rosin.

7. An antiviral composition comprising the antiviral agent according to any one of claims 1 to 6 and a solvent, the antiviral composition being used for imparting antiviral properties to a subject.

8. The antiviral composition according to claim 7, further comprising a vegetable oil.

9. The antiviral composition according to claim 7 or 8, which is used for membrane formation.

10. An antiviral membrane formed by using the antiviral composition according to claim 9.

11. An antiviral article comprising the antiviral membrane according to claim 10 on at least a part of a surface.

## INTERNATIONAL SEARCH REPORT

| International application No. |
| --- |
| **PCT/JP2022/027429** |

### A. CLASSIFICATION OF SUBJECT MATTER

*A01N 65/06*(2009.01)i; *A01N 31/02*(2006.01)i; *A01N 61/00*(2006.01)i; *A01N 63/10*(2020.01)i; *A01P 1/00*(2006.01)i;
*A61K 35/10*(2015.01)i; *A61K 36/15*(2006.01)i; *A61P 31/12*(2006.01)i
FI:    A01N65/06; A01N63/10; A01N61/00 Z; A01N31/02; A01P1/00; A61K36/15; A61K35/10; A61P31/12

According to International Patent Classification (IPC) or to both national classification and IPC

### B. FIELDS SEARCHED

Minimum documentation searched (classification system followed by classification symbols)

A01N65/06; A01N31/02; A01N61/00; A01N63/10; A01P1/00; A61K35/10; A61K36/15; A61P31/12

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII)

### C. DOCUMENTS CONSIDERED TO BE RELEVANT

| Category* | Citation of document, with indication, where appropriate, of the relevant passages | Relevant to claim No. |
| --- | --- | --- |
| X | US 2016/0051611 A1 (PROFESSIONAL COMPOUNDING CENTERS OF AMERICA (PCCA)) 25 February 2016 (2016-02-25) paragraphs [0031], [0040], [0045]-[0046] | 1, 7-11 |
| A | | 2-6 |
| X | US 2020/0397711 A1 (LEE, Gregory Brian) 24 December 2020 (2020-12-24) paragraphs [0030]-[0031], [0036] | 7-11 |
| A | | 1-6 |
| X | US 2016/0051607 A1 (PROFESSIONAL COMPOUNDING CENTERS OF AMERICA (PCCA)) 25 February 2016 (2016-02-25) claims | 7-11 |
| A | | 1-6 |

☑ Further documents are listed in the continuation of Box C.    ☑ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **12 September 2022** | **27 September 2022** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**

International application No.

**PCT/JP2022/027429**

## C. DOCUMENTS CONSIDERED TO BE RELEVANT

| Category* | Citation of document, with indication, where appropriate, of the relevant passages | Relevant to claim No. |
|---|---|---|
| X | WO 2021/023885 A1 (CAMBRIDGE ENTERPRISE LIMITED) 11 February 2021 (2021-02-11)<br>claims, p. 21, lines 17-28 | 7-11 |
| A | | 1-6 |
| X | CN 105010727 A (HEFEI LAISI BIOLOGICAL ENGINEERING CO., LTD.) 04 November 2015 (2015-11-04)<br>claims | 7-11 |
| A | | 1-6 |
| X | JP 2018-65840 A (SCHENTAG, Jerome) 26 April 2018 (2018-04-26)<br>claims, examples | 7-8 |
| A | | 1-6, 9-11 |
| X | JP 2005-526756 A (NOVARTIS AG) 08 September 2005 (2005-09-08)<br>claims | 7-8 |
| A | | 1-6, 9-11 |
| A | TEIXEIRA, Francisco Bruno. Copaiba oil-resin (Copaifera reticulata Ducke) modulates the inflammation in a model of injury to rats' tongues. BMC Complementary and Alternative Medicine. 14 June 2017, vol. 17, pp. 1-8<br>in particular, abstract | 1-11 |
| A | JP 2021-95381 A (HANIC WHITE LABO CO LTD) 24 June 2021 (2021-06-24)<br>claims | 1-11 |

Form PCT/ISA/210 (second sheet) (January 2015)

**INTERNATIONAL SEARCH REPORT**
Information on patent family members

International application No.

**PCT/JP2022/027429**

| Patent document cited in search report | | | Publication date (day/month/year) | Patent family member(s) | | | Publication date (day/month/year) |
|---|---|---|---|---|---|---|---|
| US | 2016/0051611 | A1 | 25 February 2016 | (Family: none) | | | |
| US | 2020/0397711 | A1 | 24 December 2020 | (Family: none) | | | |
| US | 2016/0051607 | A1 | 25 February 2016 | (Family: none) | | | |
| WO | 2021/023885 | A1 | 11 February 2021 | GB | 2584056 | A | |
| | | | | CN | 114514042 | A | |
| CN | 105010727 | A | 04 November 2015 | (Family: none) | | | |
| JP | 2018-65840 | A | 26 April 2018 | US | 2011/0268795 | A1 | |
| | | | | claims, examples | | | |
| | | | | WO | 2013/063527 | A1 | |
| | | | | EP | 2680859 | A1 | |
| | | | | AU | 2012223528 | A | |
| | | | | CN | 103635196 | A | |
| | | | | EA | 201391262 | A | |
| | | | | KR | 10-2014-0147657 | A | |
| | | | | AU | 2017202713 | A | |
| JP | 2005-526756 | A | 08 September 2005 | US | 2005/0169971 | A1 | |
| | | | | claims | | | |
| | | | | WO | 2003/075895 | A1 | |
| | | | | EP | 1490037 | A1 | |
| | | | | CA | 2478811 | A | |
| | | | | BR | 308378 | A | |
| | | | | NZ | 535167 | A | |
| | | | | CN | 1652754 | A | |
| JP | 2021-95381 | A | 24 June 2021 | WO | 2021/125315 | A1 | |
| | | | | claims | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019045110 A

- JP 2021186350 A